# FASCICULE DE BREVET EUROPEEN

(11) **EP 1 609 463 B1**
(45) Date de publication et mention de la délivrance du brevet: **18.05.2011**
(21) Numéro de dépôt: 05300501.3
(22) Date de dépôt: 22.06.2005
(51) Int. Cl.: A61K 8/99, A61K 8/19, A61K 8/27, A61Q 19/00

(54) **Procédé et compositions utiles pour prévenir et/ou traiter les peaux sensibles et/ou sèches**
Verfahren und Zusammensetzungen zur Verhütung und/oder zur Behandlung von empfindlicher und/oder trockener Haut
Process and compositions for the protection and/or the treatment of dry and/or sensitive skin

(30) Priorité: 23.06.2004 FR 0451317
(43) Date de publication de la demande: 28.12.2005
(73) Titulaire: L'Oréal, 75008 Paris (FR); Nestec S.A., 1800 Vevey (CH)
(72) Inventeur: Breton, Lionel, 78000, VERSAILLES (FR); Jourdain, Roland, 92360, MEUDON LA FORET (FR); Gueniche, Audrey, 92500, RUEIL MALMAISON (FR); Bureau-Franz, Isabelle, CH-1110, MORGES (CH); Blum-Sperisen, Stéphanie, CH-1801, MONT-PELERIN (CH)
(74) Mandataire: Le Coupanec, Pascale A.M.P.

(56) Documents cités:
- EP-A- 0 399 909
- EP-A- 0 737 471
- EP-A- 0 904 784
- EP-A- 0 931 543
- EP-A- 1 110 555
- EP-A- 1 236 463
- WO-A-00/70972
- WO-A-01/15715
- WO-A-01/17365
- WO-A-03/070260
- WO-A-03/071883
- FR-A- 2 733 151
- FR-A- 2 848 448
- US-A1- 2003 003 107
- US-A1- 2004 001 817
- DATABASE WPI Section Ch, Week 200441 Derwent Publications Ltd., London, GB; Class B04, AN 2004-437798 XP002312603 & RU 2 228 184 C2 (PARTNER STOCK CO) 10 mai 2004 (2004-05-10)

## Description

La présente invention concerne un procédé de traitement cosmétique ainsi qu'une composition, notamment cosmétique et/ou dermatologique, destinés plus particulièrement à la prévention et/ou au traitement des peaux qualifiées de peaux sensibles et/ou sèches.

D'une manière générale, les peaux sensibles se définissent par une réactivité particulière de la peau. Toutefois, par opposition aux peaux qualifiées d'allergiques, cette réactivité ne relève pas d'un processus immunologique c'est-à-dire ne se produit pas uniquement au niveau d'une peau déjà sensibilisée, en réponse à la présence d'un allergène. Son mécanisme est dit aspécifique.

Cette réactivité cutanée se traduit généralement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultra-violets ou infrarouges. Il existe également des facteurs associés comme l'âge et le type de peau. Ainsi les peaux sensibles sont plus fréquentes parmi les peaux sèches ou grasses que parmi les peaux normales.

L'apparition de ces signes d'inconfort, qui apparaissent dans les minutes qui suivent la mise en contact du sujet avec l'élément déclenchant, est une des caractéristiques essentielles des peaux sensibles. Il s'agit pour l'essentiel de sensations dysesthésiques. On entend par sensations dysesthésiques, des sensations plus ou moins douloureuses ressenties dans une zone cutanée comme les picotements, fourmillements, démangeaisons ou prurits, brûlures, échauffements, inconforts, tiraillements, etc. Ces signes subjectifs existent le plus souvent en l'absence de signes chimiques visibles tels que la rougeur et les desquamations. On sait aujourd'hui que ces réactions d'irritation et d'intolérance cutanée sont notamment liées à une libération de neuropeptides par les terminaisons nerveuses de l'épiderme et du derme.

Dans le document EP 737 471, il a été montré que l'incorporation de certains sels de métaux alcalino-terreux dans une composition cosmétique et/ou dermatologique permettait de s'opposer efficacement à la libération de ces neuropeptides et d'obtenir avantageusement un effet préventif et/ou curatif des peaux sensibles. Le document EP 806 933, illustre plus particulièrement l'efficacité des sels de strontium pour traiter les peaux irritables.

Toutefois, on ne dispose toujours pas à ce jour de solution totalement satisfaisante pour prévenir et/ou traiter ce type de peaux qualifiées de sensible et ce problème est plus particulièrement exacerbé dans le cas où cette peau sensible est associée à une peau sèche. La peau sèche se manifeste essentiellement par une sensation de tiraillement et/ou de tension et elle est souvent associée à une baisse du taux d'hydratation cutanée et une altération de la fonction barrière, mesurée par la perte insensible en eau.

Le document WO 02/28402 décrit pour sa part que des microorganismes probiotiques peuvent avoir également un effet bénéfique dans la régulation de réactions d'hypersensibilité cutanée comme les réactions inflammatoires et allergiques qui relèvent d'un processus immunologique par opposition à la réactivité d'une peau sensible. Il est également rapporté dans «Probiotics in the managment of atopic eczema, Clinical and Experimental Allergy 2000», Volume 30, pages 1604-1610, une étude concernant l'effet de probiotiques sur les mécanismes immunitaires infantiles comme par exemple la dermatite atopique.

Quant au document EP 1 110 555, il propose d'utiliser des agents bactériens, notamment de type probiotique pour stabiliser et/ou réguler l'écoflore cutanée. Ces composés y sont décrits comme efficaces pour prévenir l'adhésion de la flore pathogène sur la peau.

Il est également connu des documents US 2004/0001817, WO 03/071883, WO 99/49877, WO 00/49885. EP 1 364 586, WO 00/70972, EP 1 169 925, DE 202 02 562, RU 2 228 184. WO 2004/112509 et FR 2 733 151, des compositions nutritionnelles ou thérapeutiques, mettant en oeuvre des microorganismes probiotiques, tels que des bactéries à acide lactique, notamment de type *Lactobacillus,* des bifidobactéries ou du genre *Bacillus.*

FR 2 848 448 propose des compositions à administration orale, destinées à corriger les désordres associés à une peau sèche, comprenant un extrait de grains de café décaféiné, et pouvant comprendre en outre des *Lactobacillus* lyophilisés en association ou non avec des sels de magnésium et de calcium.

WO 03/070260 divulgue des compositions d'administration orale pour la photoprotection de la peau, comprenant notamment une bactérie à acide lactique et une levure.

Par ailleurs, US 2003/003107 propose des compositions topiques mettant en oeuvre un extrait de *Bacillus coagulans*, pour inhiber le développement de microorganismes pathogènes sur la peau.

Quant à EP 0 399 909. il décrit des compositions cosmétiques s'opposant au vieillissement de la peau, comprenant notamment des sels de magnésium et de potassium, et éventuellement un extrait de levure.

De manière inattendue, les inventeurs ont constaté que ces microorganismes notamment probiotiques pouvaient s'avérer efficaces, en particulier chez l'adulte, pour le traitement des peaux sensibles en particulier associées à une peau sèche sous réserve qu'ils soient associés à une quantité efficace d'au moins un cation minéral divalent.

Cette efficacité est d'autant plus inattendue que ces deux types d'actifs étaient jusqu'à ce jour présumés agir selon deux modes d'action totalement distincts et qui impliquaient généralement un mode d'administration spécifique à chacun d'eux à savoir principalement oral, pour les microorganismes notamment probiotiques et, majoritairement topique, pour les cations minéraux divalents et en particulier pour les sels de métaux alcalino-terreux. Qui plus est, rien ne laissait présager de leur efficacité vis-à-vis d'une peau sensible associée à une peau sèche.

En conséquence, la présente invention concerne selon un premier de ses aspects, un procédé de traitement cosmétique pour prévenir et/ou traiter les peaux sensibles, comprenant l'administration orale d'au moins une quantité efficace d'au moins un microorganisme, probiotique choisi parmi les *Lactobacilhus johnsonii* (CNCM I-1225), *Lactobacillus paracasei* (CNCM I-2116), *Bifidobacterium adolescentis* CNCM I-2168). *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec une quantité efficace d'au moins un cation minéral divalent.

Elle concerne également un procédé de traitement cosmétique pour prévenir et/ou traiter les peaux sèches comprenant l'administration orale, d'au moins une quantité efficaces d'au moins deux microorganismes, dont au moins un est de type probiotique et est choisi parmi *les Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec une quantité efficaces d'au moins un cation minéral divalent.

Elle concerne également un procédé de traitement cosmétique pour prévenir et/ou traiter les peaux sensibles, comprenant l'administration topique, d'au moins une quantité efficace d'au moins un microorganisme probiotique choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* CNCM I-3446), *Bifidobacterium longum* (BB536), et leurs mélanges et/ou l'une de ses fractions et/ou l'un de ses métabolites en association avec une quantité efficace d'au moins un cation minéral divalent sous la forme d'un sel différent d'un sulfate.

Elle concerne également un procédé de traitement cosmétique pour prévenir et/ou traiter les peaux sèches, comprenant l'administration topique, d'au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec une quantité efficace d'au moins un cation minéral divalent sous la forme d'un sel différent d'un sulfate.

La présente invention concerne en outre, selon un autre de ses aspect, une composition destinée à une administration orale, comprenant au moins une quantité efficace d'au moins deux microorganismes, dont au moins un est de type probiotique, et est choisi parmi les *Lactobacillus johnsonii* (CNCM I-1225), *Lactobacillus paracasei* CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges; et/ou une de ses fractions et/ou un de ses métabolites en association avec une quantité efficace d'au moins un cation minéral divalent pour pour le traitement et/ou la prévention choisies parmi la dermatite atopique et les ichtyoses.

La présente invention concerne en outre, selon un autre de ses aspects, une composition comprenant au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2174), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent sous la forme d'un sel différent d'un sulfate pour le traitement et/ou la prévention des peaux sèches choisies parmi la dermatite atopique et les ichtyoses

Selon une première variante, cette association est formulée sous la forme d'un complément alimentaire voire d'une denrée alimentaire.

Selon une seconde variante, elle se présente sous la forme d'une composition cosmétique et/ou dermatologique selon l'invention.

La présente invention vise également l'utilisation cosmétique par voie orale, comme agent destiné à prévenir et/ou traiter les peaux sensibles d'une quantité efficace d'au moins un microorganisme probiotique choisi parmi les *Lactobacillus johnsonii* (CNCM I-1225), *Lactobacillus paracasei* GNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent.

La présente invention vise également l'utilisation cosmétique comme agent destiné à prévenir et/ou traiter les peaux sensibles, d'au moins une quantité efficace d'au moins un microorganisme probiotique choisi parmi *les Lactobacillus johnsonii* (CNCM I-1225), *Lactobacillus paracasei* (CNCM I-2116), *Bifidobacterium adolescentis* (CNCM I-2168). *Bifidobacterium longum* (CNCM I-2170), *Bifidobacterium lactis* (CNCM *I-3446), Bifidobacterium longum* (BB536), et leurs mélanges et/ou l'une de ses fractions et/ou l'un de ses métabolites en association à une quantité efficace d'au moins un cation minéral divalent sous la forme d'un sel différent d'un sulfate.

Elle vise également l'utilisation cosmétique par voie orale comme agent destiné à prévenir et/ou traiter les peaux sèches d'une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent.

Elle vise également l'utilisation cosmétique comme agent destiné à prévenir et/ou traiter les peaux sèches d'au moins une quantité efficaces d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-*3446),_Bifidobacterium longum* (BB536), et leurs mélanges et/ou l'une de ses fractions et/ou l'un de ses métabolites en association à une quantité efficace d'au moins un cation minéral divalent sous la forme d'un sel différent d'un sulfate.

Selon un autre de ses aspects, la présente invention concerne une composition cosmétique et/ou dermatologique, utile pour prévenir et/ou traiter les peaux sensibles et/ou sèches, comprenant dans un support physiologiquement acceptable au moins une quantité efficace d'au moins deux microorganismes probiotiques choisis parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins deux métaux alcalinoterreux sous la forme de sels organiques, anhydres ou hydratés ou de complexes chélatés.

La présente invention concerne selon un autre de ses aspects, une composition cosmétique et/ou dermatologique, utile pour prévenir et/ou traiter les peaux sensibles et/ou sèches, comprenant dans un support physiologiquement acceptable au moins une quantité efficace d'au moins deux microorganismes probiotiques choisis parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins un métal alcalinoterreux sous la forme d'un sel choisi parmi des bicarbonates, des glycérophosphates, des nitrates, des acétates, des hydroxydes, des sels d'α-hydroxyacides comme les citrates, tartrates, lactates et malates ou d'acides de fruits, des sels d'acides aminés comme les aspartates, arginates, et fumarates ou des sels d'acides gras comme les palmitates, oléates, caséinates et béhénates.

### Peau sensible et/ou sèche

Comme précisé précédemment, une peau sensible est différente d'une peau allergique. Sa réactivité ne relève pas d'un processus immunologique et se traduit généralement uniquement par des sensations dysesthétiques.

Pour des raisons évidentes, l'absence de signes visibles rend difficile le diagnostic de peau sensible. Le plus souvent ce diagnostic repose sur l'interrogatoire du patient. Cette symptomatologie a en outre pour intérêt de permettre de différencier la peau sensible associée ou non à une peau sèche, de l'irritation ou de l'allergie de contact pour lesquelles il existe en revanche des signes inflammatoires visibles.

En conséquence, la mise au point de produits « peaux sensibles » a nécessité de disposer d'outils d'évaluation de la réaction sensorielle de la peau. Les premiers outils se sont inspirés dès leur conception de la caractéristique essentielle des peaux sensibles à savoir présence de signes d'inconfort induits par une application topique. Ainsi, le « stinging test » à l'acide lactique a été le premier test proposé. Il est réalisé par relevé des sensations de picotements rapportées par un volontaire après application d'une solution d'acide lactique à 10 % sur les ailes du nez. Les sujets rapportant des sensations modérées ou fortes de picotements sont appelées « stingers » et considérés comme étant à peau sensible. En raison de cette sensibilité cutanée à l'application topique de produit, ces sujets sont alors sélectionnés pour tester des produits dits peaux sensibles. Plus récemment, pour activer spécifiquement les terminaisons nerveuses périphériques, impliquées dans l'inconfort et appelées nocicepteurs, récemment identifiées comme étant impliquées dans la peau sensible, de nouveaux tests ont été proposés qui utilisent précisément d'autres inducteurs d'inconfort comme la capsaïcine.

Ce second type de test, décrit dans la demande EP 1 374 913, constitue également un autre outil particulièrement utile pour le diagnostic de peaux sensibles.

Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-à-dire par des démangeaisons ou par des picotements, à différents facteurs tels que l'environnement, les émotions, les aliments, le vent, les frottements, le rasoir, l'eau dure à forte concentration de calcaire, les variations de température ou la laine.

D'une manière générale, ces deux types de peau peuvent être associés à une sécheresse cutanée avec ou sans dartres ou à une peau qui présente un érythème.

Comme précisé précédemment, la sécheresse cutanée est souvent associée à une baisse du taux d'hydratation cutanée, évalué par cornéométrie et à une altération de la fonction barrière, mesurée par la perte insensible en eau.

La peau sèche se manifeste essentiellement par une sensation de tiraillements et/ou de tension. Celle-ci est aussi rugueuse au toucher et apparaît couverte de squames. Lorsque la peau est légèrement sèche, ces squames sont abondantes mais peu visibles à l'oeil nu. Elles sont de moins en moins nombreuses mais de plus en plus visibles à l'oeil nu lorsque ce désordre s'aggrave.

L'origine de cette sécheresse cutanée peut être de type constitutionnel ou acquis.

Dans le cas de peau sèche acquise, l'intervention de paramètres extérieurs tels que l'exposition aux agents chimiques, à des conditions climatiques difficiles, aux rayons solaires ou bien encore certains traitements thérapeutiques (rétinoïdes, par exemple) est déterminante. Sous ces influences extérieures, la peau peut devenir alors momentanément et localement sèche. Cela peut concerner tout type de peau, normal et même gras.

Dans le cas de la peau sèche constitutionnelle, on peut distinguer deux catégories : les peaux pathologiques et les peaux non pathologiques.

Les peaux sèches constitutionnelles pathologiques sont essentiellement représentées par la dermatite atopique et les ichthyoses. Elles sont quasiment indépendantes des conditions extérieures.

La dermatite atopique est décrite comme associée à un déficit dans le métabolisme des lipides du stratum corneum et notamment des céramides. Cette pathologie se présente sous la forme d'une xérose plus ou moins chronique concernant une grande étendue du corps, associée à des poussées inflammatoires et prurigineuses par plaques.

Les ichthyoses sont des pathologies caractérisées par un déficit génétique affectant le processus de kératinisation à différents stades. Elles se manifestent par une desquamation importante par plaques.

Les peaux sèches constitutionnelles non pathologiques sont des peaux sèches dont la sévérité peut dépendre des facteurs extérieurs déjà évoqués. Rentrent dans cette catégorie de peau, la peau sénile (caractérisée par une diminution générale du métabolisme cutané avec l'âge), la peau fragile (très sensible aux facteurs extérieurs et souvent accompagnée d'érythème et de rosacée) et la xérose vulgaire (d'origine génétique probable et se manifestant en priorité sur le visage, les membres et le dos des mains).

Les compositions et procédé selon l'invention, s'avèrent ainsi tout particulièrement efficaces pour prévenir et/ou traiter les peaux sensibles et/ou sèches et plus particulièrement les peaux dites réactives, irritables et/ou intolérantes, les peaux sèches acquises et/ou les peaux sèches constitutionnelles.

### Microorganismes et notamment microorganismes probiotiques

Les microorganismes convenant à l'invention sont des microorganismes qui peuvent être administrés sans risques à l'animal ou l'homme.

En particulier, on utilise dans la présente invention au moins un microorganisme dit de type probiotique, et choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites.

Au sens de la présente invention, on entend par « microorganisme probiotique », un microorganisme vivant qui, lorsqu'il est consommé en quantité adéquate, a un effet positif sur la santé de son hôte « joint FAO/WHO Expert Consultation on Evaluation of Health and Nutritional Properties of Probiotic in Food Including Powder Milk with Live Lactic Acid Bacteria, 6 octobre 2001 », et qui peut en particulier améliorer l'équilibre microbien intestinal.

Au sens de l'invention, le terme métabolite désigne toute substance issue du métabolisme des microorganismes considérés selon l'invention et dotée également d'une efficacité pour le traitement des peaux sensibles.

Au sens de l'invention, le terme fraction désigne plus particulièrement un fragment dudit microorganisme doté d'une efficacité pour le traitement des peaux sèches par analogie audit microorganisme entier.

Selon un mode de réalisation particulier de l'invention, une composition peut comprendre au moins deux microorganismes, notamment probiotiques et/ou métabolites et/ou fractions différents. Ces microorganismes peuvent différer par leur nature par exemple bactérie et champignon, ou bien encore par leur famille, leur genre, leur espèce, ou seulement par leur souche.

Une composition selon l'invention peut ainsi comprendre au moins un microorganisme choisi parmi ceux cités précédemment, et un second microorganisme choisi ou non parmi les microorganismes précités.

Les microorganismes convenant à l'invention peuvent être choisis notamment parmi les ascomycetes telles que *Saccharomyces, Yarrowia, Kluyveromyces, Torulaspora, Schizosaccharomyces pombe, Debaromyces, Candida, Pichia, Aspergillus* et *Penicillium,* des bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus* et leurs mélanges.

Comme ascomycetes convenant tout particulièrement à la présente invention, on peut en particulier citer *Yarrowia lipolitica et Kluyveromyces lactis,* de même que *Saccharomyces cereviseae, Torulaspora, Schizosaccharamyces pombe, Candida* et *Pichia.*

En ce qui concerne les microorganismes probiotiques, ce sont les genres bactériens et de levure suivants qui sont généralement utilisés :
- les bactéries lactiques : qui produisent par fermentation du sucre de l'acide lactique. Suivant leur morphologies ont les divisent en deux groupes :
   - *Lacobacillus species : acidophilus (LCI, NCFB 1748*); *amylovorus, casei (Shirota), rhamnosus* (soude GG), *brevis, crispatus, delbrueckii (subsp bulgaricus, lactis), fermemtum, helveticus, gallinarum, gasseri, lohnsonii, paracasei, plantarum, reuteri, rhamnosus, salivarius),*
   - *Gocci : Enterocossus (faecalis, faeciul), Lactococcus lactis (subspp lactis ou cremoris), Leuconstoc mesenteroides* subsp *dextranicum, Pediococcus acidilactici* (alimentation animale), *Sporolactobacillus inulinus, Streptococcus salvarius subsp. Thermophilus*
- Les bifidobactéries ou *Bifidobacterium species: Bifidobacterium adolescentes; animalis, bifidum, breve, lactis, longum, infantis,*
- Les levures : *Saccharomyces (cerevisiae* ou encore *boulardii),*
- Les autres bactéries sporulées : *Bacillus (cereus var toyo ou subtilis), Bacillus coagulans, B licheniformis, Escherichia colis train nissle, Propionibacterium freudenreichii,*

Les bactéries lactiques et les bifidobactéries sont les probiotiques le plus souvent utilisés.

Des exemples spécifiques de microorganismes probiotiques convenant tout particulièrement à l'invention sont *Bifidobacterium bifidum, Bifidobacterium infantis, Bifidobacterium longum, Lactobacillus acidophilus, Lactobacillus alimentarius, Lactobacillus casei subsp. Casei, Lactobacillus casei Shirota, Lactobacillus paracasei, Lactobacillus curvatus, Lactobacillus delbruckii subsp. Lactis, Lactobacillus gasseri, Lactobacillus johnsonii, Lactobacillus reuteri, Lactobacillus rhamnosus (Lactobacillus GG), Lactobacillus sake, Lactococcus lactis, Streptococcus thermophilus, Staphylococccus carnosus,* et *Staphylococcus xylosus et leurs mélanges.*

Les microorganismes peuvent être formulés à l'état de poudres, c'est-à-dire sous une forme sèche, ou sous forme de suspensions ou de solutions.

Si nécessaire, ces microorganismes peuvent être formulés au sein des compositions selon l'invention sous une forme encapsulée de manière à améliorer significativement leur durée de survie. Dans un tel cas, la présence d'une capsule peut en particulier retarder ou éviter la dégradation du microorganisme au niveau du tractus gastro intestinal.

Plus particulièrement, il s'agit de microorganismes probiotiques issus du groupe des bactéries lactiques, comme notamment les *Lactobacillus et*/*ou* les *Bifidobacterium.* A titre illustratif de ces bactéries lactiques, on peut plus particulièrement citer les *Lactobacillus johnsonii, Lactobacillus reuteri, Lactobacillus rhamnosus, Lactobacillus paracasei, Lactobacillus casei* ou *Bifidobacterium bifidum, Bifidobacterium breve, Bifidobacterium longum, Bifidobacterium animalis, Bifidobacterium lactis, Bifidobacterium infantis, Bifidobacterium adolescentis* ou *Bifidobacterium pseudocatenulatum* et leurs mélanges.

Les espèces convenant tout particulièrement sont les *Lactobacillus johnsonii, Lactobacillus paracasei, Bifidobacterium adolescentis, Bifidobacterium longum* et *Bifidobacterum, Lactis NCC 2818 (encore désigné Bb12 ATCC 27536)* respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168 et CNCM 1-2170 et CNCM I-3446, et le genre *Bifidobacterium longum (BB536).* La souche de *Bifidobacterium lactis* CNCM I-3446 peut être obtenue chez Hansen (Chr. Hansen A/S, 10-12 Boege Alle, P.O. Box 407, DK-2970 Hoersholm, Danemark).

Selon une variante de l'invention, la composition contient au moins un microorganisme *Lactobacillus sp* et au moins un microorganisme *Bifidobacterium sp,* notamment dans des quantités équivalentes et plus particulièrement à raison de 10¹⁰ ufc respectivement.

Les microorganismes et/ou leurs fractions et/ou métabolites peuvent être formulés dans un support approprié dans une quantité d'au moins 10³ ufc/g, en particulier à des doses variant de 10⁵ à 10¹⁵ ufc/g, et plus particulièrement de 10⁷ à 10¹² ufc/g de support.

D'une manière générale, les compositions selon l'invention et en particulier celles destinées à être administrées par voie orale peuvent comprendre pour les microorganismes vivants de 10³ à 10¹⁵ ufc/g, en particulier de 10⁵ à 10¹⁵ ufc/g et plus particulièrement de 10⁷ à 10¹² ufc/g de microorganismes par gramme de support ou à des doses équivalentes calculées pour les microorganismes inactifs ou morts ou pour des fractions de microorganisme ou pour des métabolites produits. Les compositions à application topique selon l'invention comprennent généralement de 10³ à 10¹² ufc/g, en particulier de 10⁵ à 10¹⁰ ufc/g et plus particulièrement de 10⁷ à 10⁹ ufc/g de microorganismes notamment probiotiques.

Lorsque la composition comprend des métabolites, les teneurs en métabolites dans les compositions correspondent sensiblement aux teneurs susceptibles d'être produites par 10³ à 10¹⁵ ufc, en particulier 10⁵ à 10¹⁵ ufc, et plus particulièrement 10⁷ à 10¹² ufc de microorganismes vivants par gramme de support.

Dans le cas particulier des compositions devant être administrées par voie orale, la concentration en microorganisme(s) notamment probiotique(s) peut être ajustée de manière à correspondre à des doses (exprimées en équivalent de microorganisme) variant de 5.10⁵ à 10¹³ ufc/j et en particulier de 10⁸ à 10¹¹ ufc/j.

Le ou les microorganisme(s) peu(ven)t être inclus dans la composition selon l'invention sous une forme vivante, semi-active ou inactivée, morte.

Il(s) peu(ven)t également être inclus sous forme de fractions de composants cellulaires ou sous la forme de métabolites. Le ou le(s) microorganisme(s), métabolite(s) ou fraction(s) peu(ven)t également être introduit(s) sous la forme d'une poudre lyophilisée, d'un surnageant de culture et/ou le cas échéant sous une forme concentrée.

Dans le cas particulier des compositions topiques, il peut être avantageux de mettre en oeuvre ces microorganismes sous forme inactivée voire morte.

### Cation minéral divalent

Selon l'invention, on peut utiliser un ou plusieurs cation(s) minéral(aux) divalent(s).

En particulier, on utilise dans la présente invention au moins deux voire trois cations minéraux divalents différents.

Dans le cadre de la présente invention, on peut utiliser les cations minéraux divalents sous différentes formes. Le cation minéral divalent peut ainsi être sous la forme d'un sel minéral ou organique, anhydre ou hydraté ou d'un complexe chelaté.

Ces sels peuvent être par exemple des carbonates, des bicarbonates, des sulfates, des glycérophosphates, des chlorures, des nitrates, des acétates, des hydroxydes, des oxydes, des sels d'α-hydroxyacides (citrates, tartrates, lactates, malates) ou d'acides de fruits, ou encore des sels d'acides aminés (aspartate, arginate, fumarate) ou des sels d'acides gras (palmitate, oléate, caséinate, béhénate).

Selon un mode de réalisation particulier, le cation minéral divalent est choisi parmi le manganèse, le cuivre et/ou le zinc.

Selon un autre mode de réalisation particulier, le cation minéral divalent est un métal alcalino-terreux. Comme métal alcalino-terreux utilisable dans l'invention, on peut citer le baryum, le calcium, le magnésium, le strontium et/ou le béryllium.

Avantageusement, le cation minéral divalent et notamment métal alcalino-terreux est utilisé dans la présente invention sous forme de sel. En particulier, le sel peut être choisi parmi le nitrate de calcium, le nitrate de strontium, le gluconate de magnésium, le lactate de calcium, le gluconate de strontium, le lactate de magnésium, le chlorure de calcium, le chlorure de strontium, le chlorure de magnésium, le carbonate de calcium, le sulfate de strontium, le sulfate de magnésium, le glycérophosphate de calcium, le citrate de calcium, le citrate de magnésium, l'acétate de strontium, l'acétate de magnésium et leurs mélanges.

Selon un mode de réalisation particulièrement avantageux, on utilise au moins un cation minéral divalent choisi parmi les sels de citrate, chlorure, gluconate, sulfate, lactate et/ou acétate, de strontium, de calcium et/ou de magnésium et leurs mélanges.

Selon un mode de réalisation, les procédés utilisés et compositions selon l'invention mettent en oeuvre au moins deux cations minéraux divalents et en particulier deux métaux alcalno-terreux notamment différents sous la forme de sels organiques.

Le cation minéral divalent peut aussi être utilisé sous la forme d'un complexe chélaté notamment à des protéines cristallisées ou ionisées.

Le cation minéral divalent peut encore être sous une forme spécifique stockée par un microorganisme, par exemple de type levure, à l'image des levures séléniées.

Ainsi, les cations peuvent être introduits tels quels dans les compositions selon l'invention ou par le biais d'un composé ou mélange de composé(s), connus pour contenir au moins l'un de ces cations en une concentration élevée. Par exemple, comme source de sels métalliques, on peut utiliser un extrait de plantes ou levures enrichis en cations. De même, le calcium peut par exemple être introduit par l'intermédiaire d'un extrait lacté.

La teneur en cation minéral divalent utilisée dans les compositions selon l'invention dépend bien entendu de la forme du cation considéré et peut être déterminée à l'aide de simples expériences de routine. Ces doses quotidiennes peuvent en particulier aller de 100 µg à 5 g, plus particulièrement de 1 mg à 2 g, voire de 10 mg à 1,3 g.

Dans les compositions destinées à une administration orale selon l'invention, la concentration en cation minéral divalent peut être ajustée de manière à correspondre à des doses variant de 1 à 3000 mg/jour et en particulier de 10 à 2000 mg/jour.

A titre illustratif des compositions et procédés conformes à l'invention, on peut notamment citer celles associant au moins un sel de magnésium et/ou de calcium, notamment organique et en particulier choisi parmi du gluconate, du lactate et/ou de citrate de magnésium et/ou de calcium et/ou le nitrate de strontium, avec au moins une bactérie lactique notamment choisie parmi les *Lactobacillus sp* et plus particulièrement la *Bifidobacterium lactis* dite CNCM I-3446.

Selon les variantes de l'invention, les compositions peuvent être administrées par voie topique ou par voie orale.

Les compositions selon l'invention peuvent se présenter sous toutes les formes galéniques normalement utilisées selon la voie d'utilisation.

Le support peut être de nature diverse selon le type de composition considérée.

Conviennent notamment comme supports alimentaires ou pharmaceutiques, le lait, le yaourt, le fromage, les laits fermentés, les produits fermentés à base de lait, des glaces, des produits à base de céréales fermentées, des poudres à base de lait, des formules pour enfants et nourrissons, des aliments pour animaux en particulier domestiques, des comprimés ou tablettes, des suspensions de bactéries liquides, des suppléments oraux sous forme sèche et les suppléments oraux sous forme liquide.

En particulier, la composition selon l'invention peut être une composition alimentaire pour la consommation humaine. Il peut s'agir en particulier d'aliments complets nutritionnels, de boissons, d'eaux minérales, de soupes, de suppléments diététiques et d'aliments de remplacement, de barres nutritionnelles, de confiserie, de produits à base de lait ou à base de lait fermenté, de yaourts, de poudres à base de lait, de produits de nutrition entérale, de compositions pour enfants et/ou nourrissons, de produits à base de céréales ou de produits à base de céréales fermentées, de glaces, de chocolat, de café, de produits « culinaires » tels que de la mayonnaise, de la purée de tomate ou des assaisonnements pour salades. La composition selon l'invention peut également être destinée aux animaux.

En ce qui concerne plus particulièrement les produits cosmétiques, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique.

Pour l'ingestion, de nombreuses formes de réalisation de compositions orales et notamment de compléments alimentaires sont possibles. Leur formulation est réalisée par les procédés usuels pour produire des dragées, gélules, gels, émulsions, comprimés, capsules ou solutions. En particulier, le(s) actif(s) selon l'invention peuvent être incorporés dans toutes autres formes de compléments alimentaires ou d'aliments enrichis, par exemple des barres alimentaires, ou des poudres compactées ou non. Les poudres peuvent être diluées à l'eau, dans du soda, des produits laitiers ou dérivés du soja, ou être incorporées dans des barres alimentaires.

Les actifs selon l'invention peuvent être formulés avec les excipients et composants usuels pour de telles compositions orales ou compléments alimentaires, à savoir notamment composants gras et/ou aqueux, agents humectants, épaississants, conservateurs, agents de texture, de saveur et/ou d'enrobage, antioxydants, conservateurs et colorants usuels dans le domaine de l'alimentaire.

Les agents de formulation et excipients pour composition orale, et notamment pour compléments alimentaires sont connus dans ce domaine et ne font pas ici l'objet d'une description détaillée.

Bien entendu, les compositions orales selon l'invention peuvent contenir plusieurs autres actifs.

A titre d'actifs utilisables, on peut citer, les vitamines B3, B5, B6, B8, C, E, ou PP, les caroténoïdes, les curcuminoïdes et la niacine.

En particulier, on peut utiliser un complexe anti-oxydant comprenant les vitamines C et E, et au moins un caroténoïde, notamment un caroténoïde choisi parmi le β-carotène, le lycopène, l'astaxanthine, la zéaxanthine et la lutéine, des flavonoïdes telles que les catéchines, l'hespéridine, des proanthocyanidines et des anthocyanines.

La composition comprend avantageusement au moins un prébiotique ou un mélange de prébiotiques. Plus particulièrement, ces prébiotiques peuvent être choisis parmi les oligosaccharides, produits à partir du glucose, galactose, xylose, maltose, sucrose, lactose, amidon, xylane, l'hémicellulose, l'inuline, des gommes, de type acacia par exemple, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Les compositions cosmétiques et/ou dermatologiques, plus particulièrement concernées par une application topique, peuvent se présenter notamment sous forme de solutions aqueuses, hydroalcooliques ou huileuses, de dispersions du type des solutions ou dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, de gel aqueux ou anhydre, ou encore de microémulsions, de microcapsules, de microparticules, ou de dispersions vésiculaires de type ionique et/ou non ionique.

Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, ou des compositions contre les piqûres d'insectes.

Les compositions selon l'invention peuvent également consister en des préparations solides constituant des savons ou des pains de nettoyage.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique. L'émulsionnant et le coémulsionnant peuvent être présents, dans la composition, en une proportion allant de 0,3 à 30 % en poids, et de préférence de 0,5 à 20 % en poids par rapport au poids total de la composition.

Lorsque la composition de l'invention est une solution ou un gel huileux, la phase grasse peut représenter plus de 90 % du poids total de la composition.

De façon connue, la composition cosmétique et/ou dermatologique de l'invention peut contenir également des adjuvants habituels dans le domaine cosmétique pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composés siliconés comme les huiles siliconées et par exemple les cyclométhicone et diméthicone, les cires, les résines et les gommes siliconées. Ces composés peuvent être fonctionnalisés ou non.

Comme émulsionnants utilisables dans l'invention, on peut citer par exemple le stéarate de glycérol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20OE).

Comme solvants utilisables dans l'invention, on peut citer les alcools inférieurs, notamment l'éthanol et l'isopropanol, le propylène glycol.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Comme actifs hydrophiles, on peut utiliser les protéines ou les hydrolysats de protéine, les acides aminés, les polyols notamment en C₂ à C₁₀ comme les glycérine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Comme actifs lipophiles, on peut utiliser le rétinol (vitamine A) et ses dérivés, le tocophérol (vitamine E) et ses dérivés, les céramides, les huiles essentielles.

On peut, en outre, associer les actifs selon l'invention, à des agents actifs destinés notamment à la prévention et/ou au traitement des affections cutanées.

En outre, la composition de l'invention peut contenir de façon avantageuse une eau thermale et/ou minérale, notamment choisie parmi l'eau de Vittel, les eaux du bassin de Vichy et l'eau de la Roche Posay.

Le procédé de traitement cosmétique de l'invention peut être mis en oeuvre notamment en appliquant les compositions cosmétiques et/ou dermatologiques telles que définies ci-dessus, selon la technique d'utilisation habituelle de ces compositions. Par exemple : applications de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Le procédé cosmétique selon l'invention peut être mis en oeuvre par administration topique ou par une prise orale, journalière par exemple, de l'association selon l'invention qui peut être par exemple formulée sous forme de gélules, gels, lotions, dragées, émulsions, comprimés, capsules ou ampoules buvables, en quantité et nombre adéquats, selon leur forme, pour que les actifs soient administrés à raison de 5.10⁵ à 10¹³ ufc par jour, en particulier 10⁶ à 10¹¹ ufc par jour, en microorganismes ou à des doses équivalentes en microorganismes partiellement inactivés ou morts ou en fractions de microorganismes ou en métabolites produits.

Selon un autre mode de réalisation, l'administration est répétée jusqu'à ce que le cation minéral divalent soit administré à des doses de l'ordre de 1 à 3000 mg par jour, et en particulier de 10 à 2000 mg par jour.

Le procédé selon l'invention peut comprendre une administration unique. Selon un autre mode de réalisation, l'administration est répétée par exemple 2 à 3 fois quotidiennement sur une journée ou plus et généralement sur une durée prolongée d'au moins 4 semaines, voire 4 à 15 semaines, avec le cas échéant une ou plusieurs périodes d'interruption.

Dans la description et dans les exemples suivants, sauf indication contraire, les pourcentages sont des pourcentages en poids et les plages de valeurs libellées sous la forme « entre ... et ... » incluent les bornes inférieure et supérieure précisées. Les ingrédients sont mélangés, avant leur mise en forme, dans l'ordre et dans des conditions facilement déterminées par l'homme de l'art.

Les exemples ci-après sont présentés à titre illustratif et non limitatif du domaine de l'invention.

### Exemples hors invention de composition pour la voie topique

### Exemple 1 : Lotion pour le visage des peaux sensibles

| | |
|---|---|
| Poudre de *Lactobacillus sp.* | 5,00 |
| Gluconate de magnésium | 3,00 |
| Lactate de calcium | 2,00 |
| Antioxydant | 0,05 |
| Isopropanol | 40,0 |
| Conservateur | 0,30 |
| Eau | qsp 100 % |

### Exemple 2 : Lait pour le soin du visage des peaux sèches et sensibles

| | |
|---|---|
| Chlorure de magnésium | 3,00 |
| Ascorbate de calcium | 3,00 |
| Poudre de *Lactobacillus sp.* | 5,00 |
| Stéarate de glycérol | 1,00 |
| Alcool cétylstéarylique/alcool cétylstéarylique oxyéthyléné à 33 moles OE (Sinnowax AO® vendu par la société Henkel) | 3,00 |
| Alcool cétylique | 1,00 |
| Diméthicone (DC 200 Fluid® vendu par la société Dow Corning) | 1,00 |
| Huile de vaseline | 6,00 |
| Myristate d'isopropyle (Estol IMP 1514® vendu par Unichema) | 3,00 |
| Antioxydant | 0,05 |
| Glycérine | 20,00 |
| Conservateur | 0,30 |
| Eau | qsp 100 |

### Exemple 3 : Gel pour le soin du visage des peaux sensibles

| | |
|---|---|
| Nitrate de strontium | 4,00 |
| *Lactobacillus sp.* | 5,00 |
| Hydroxypropylcellulose (Klucel H® vendu par la société Hercules) | 1,00 |
| Vitamine E | 2,50 |
| Antioxydant | 0,05 |
| Isopropanol | 40,00 |
| Conservateur | 0,30 |
| Eau | qsp 100 % |

### Exemple 4 : Lait pour le soin du visage des peaux sèches et sensibles

| | |
|---|---|
| Ascorbate de magnésium | 3,00 |
| Huile de pépin de cassis | 4,00 |
| Huile de bourrache | 4,00 |
| *Lactobacillus sp.* | 5,00 |
| Stéarate de glycérol | 1,00 |
| Alcool cétylstéarylique/alcool cétylstéarylique oxyéthyléné | |
| à 33 moles OE (Sinnowax AO® vendu par la société Henkel) | 3,00 |
| Alcool cétylique | 1,00 |
| Diméthicone (DC 200 Fluid® vendu par la société Dow Corning) | 1,00 |
| Huile de vaseline | 6,00 |
| Myristate d'isopropyle (Estol IPM 1514® vendu par Unichema) | 3,00 |
| Glycérine | 20,00 |
| Conservateur | 0,30 |
| Eau | qsp 100 |

### Exemples hors invention de compositions pour la voie orale

### Exemple 5 : Stick poudre

| **Principe actif** | |
|---|---|
| *Lactobacillus sp.* | 10¹⁰ ufc |
| Citrate de magnésium | 200 mg |
| Citrate de calcium | 600 mg |
| | |

| **Excipient** | |
|---|---|
| Gomme de xanthane | 0,8 mg |
| Benzoate de sodium | 0,2 mg |
| Maltodextrine | qsp 30g |

| | |
|---|---|
| On peut prendre un stick par jour. | |

### Exemple 6 : Stick poudre

| **Principe actif** | |
|---|---|
| *Lactobacillus sp.* | 10¹⁰ ufc |
| *Bifidobacterium sp.* | 10¹⁰ ufc |
| Citrate de calcium | 50 mg |
| | |

| **Excipient** | |
|---|---|
| Gomme de xanthane | 0,8 mg |
| Benzoate de sodium | 0,2 mg |
| Maltodextrine | qsp 30g |

| | |
|---|---|
| On peut prendre un stick par jour. | |

### Exemple 7 : Stick poudre

| **Principe actif** | |
|---|---|
| *Lactobacillus sp.* | 10¹⁰ ufc |
| *Bifidobacterium sp.* | 10¹⁰ ufc |
| Citrate de magnésium | 50 mg |
| | |

| **Excipient** | |
|---|---|
| Gomme de xanthane | 0,8 mg |
| Benzoate de sodium | 0,2 mg |
| Maltodextrine | qsp 30g |

| | |
|---|---|
| On peut prendre un stick par jour. | |

### Exemple 8 : capsule

| | mg/capsule |
|---|---|
| *Lactobacillus sp.* | 10⁸ ufc |
| Gluconate de magnésium | 150 |
| Vitamine C | 60 |
| Stéarate de magnésium | 0,02 |

| | |
|---|---|
| On peut prendre une à trois de ces capsules par jour. | |

### Exemple 9 : capsule

| | mg/capsule |
|---|---|
| *Lactobacillus sp.* | 10⁹ ufc |
| Citrate de calcium | 300 |
| Vitamine C | 60 |
| Stéarate de magnésium | 0,02 |

| | |
|---|---|
| On peut prendre une à trois capsules par jour. | |

### Exemple 10 : formulation de type dragée

| **Matières actives** | mg/dragée |
|---|---|
| Gluconate de magnésium | 50 |
| *Lactobacillus sp.* | 5.10⁸ ufc |
| Citrate de calcium | 200 |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |

| **Agent d'enrobage** | |
|---|---|
| Gomme laque | 5 |
| Talc | 61 |
| Saccharose | 250 |
| Polyvidone | 6 |
| Dioxyde de titane | 0,3 |
| Agent de coloration | 5 |

| | |
|---|---|
| Ce type de dragée peut être pris 1 à 3 fois par jour. | |

### Exemple 11 : formulation de type dragée

| **Matières actives** | mg/dragée |
|---|---|
| Lactate de magnésium | 50 |
| *Bifidobacterium sp.* | 10⁹ ufc |
| *Lactobacillus sp.* | 10⁹ ufc |
| Lactate de calcium | 200 |

| **Excipient du noyau de la dragée** | |
|---|---|
| Cellulose micro-cristalline | 70 |
| Encompress™ | 60 |
| Stéarate de magnésium | 3 |
| Silice colloïdale anhydre | 1 |
| | |

| **Agent d'enrobage** | |
|---|---|
| Gomme laque | 5 |
| Talc | 61 |
| Saccharose | 250 |
| Polyvinylidone | 6 |
| Dioxyde de titane | 0,3 |
| Agent de coloration | 5 |

| | |
|---|---|
| Ce type de dragée peut être pris 1 à 3 fois par jour. | |

### Exemple de compositions pour la voie orale.

### Exemple 12

Deux compositions orales, l'une à base uniquement de microorganisme probiotique (B) et l'autre associant à ce microorganisme deux sels de métaux alcalino-terreux (C) ont été testés pour leur efficacité vis-à-vis de la sécheresse et sensibilité cutanée au regard d'une composition placebo (A). Leur composition est la suivante :
**A :** Maltodextrine.
**B :** 1x10¹⁰ ufc *Lactobacillus paracaseï CNCM I-2116 +* 1x10¹⁰ ufc *Bifidobacterium lactis* CNCM I-3446
**C :** 1x10¹⁰ ufc *Lactobacillus paracaseï CNCM I-2116 +* 1x10¹⁰ ufc *Bifidobacterium lactis* CNCM I-3446 + 1 g de citrate de calcium + 300 mg de citrate de magnésium.

Le traitement consiste à administrer quotidiennement et par voie orale une unique unité de traitement pendant une durée de huit semaines.

Cette étude a été réalisée sur 99 sujets adultes de sexe féminin dont l'âge est compris entre 18 et 50 ans, et qui ont été identifiés à la suite d'une évaluation clinique (score clinique de la sécheresse des jambes et de la rugosité du visage) et d'une auto-évaluation par questionnaire (questionnaire peau sensible validée) comme des sujets à peaux sèches et sensibles.

Ces 99 sujets ont été répartis en 3 groupes parallèles de 33 sujets, avec 2 groupes recevant les produits testés et 1 groupe recevant le placebo.

L'effet des deux compléments testés est apprécié par comparaison à la formulation témoin dite placebo. Les résultats obtenus figurent dans le tableau I ci-après.

**Tableau I**

| % de variation entre J1 et J57 et significativité *versus* placebo(1) | Complément alimentaire à base uniquement de probiotiques (B) | Complément alimentaire selon l'invention (C) |
|---|---|---|
| Score clinique : Diminution par rapport à J1 Sécheresse des jambes | - 34 % | - 42 % (p = 0,2) |
| Auto-évaluation : Diminution par rapport à J1 Sécheresse des jambes | - 28 % (p = 0,2) | - 36 % (p = 0,006) |
| Facteur d'hydratation : Augmentation par rapport à J1 Urée | +29 % (p = 0,6) | + 75 % (p = 0,02) |

| | | |
|---|---|---|
| (1) Analyse des contrastes entre J1 et J57 entre les groupes de traitement et le groupe placebo. | | |

### Exemple de composition pour la voie orale

### Exemple 13

La composition orale conforme à l'invention de l'exemple précédent à été testée en terme de sensibilité cutanée chez les sujets considérés pour l'étude de l'exemple 10 (évaluation de la sensibilité cutanée par un test à l'acide lactique ou stinging test).

Il a ainsi été constaté une réduction de la sensibilité cutanée d'environ - 42 % (p = 0,6) entre J1 et J57 chez les sujets traités.

## Revendications

1. Procédé de traitement cosmétique pour prévenir et/ou traiter les peaux sensibles, comprenant l'administration orale, d'au moins une quantité efficace d'au moins un microorganisme probiotique choisi parmi les *Lactobacillus johnsonii* (CNCM I-1225), *Lactobacillus paracasei* (CNCM I-2116), *Bifidobacterium adolescentis* (CNCM 1-2168), *Bifidobacterium longum* (CNCM- I-2170), *Bifidobacterium lactis* (CNCM 1-3446), *Bifidobacterium longum* (BB536), et leurs mélanges et/ou l'une de ses fractions et/ou l'un de ses métabolites en association avec une quantité efficace d'au moins un cation minéral divalent.

2. Procédé de traitement cosmétique pour prévenir et/ou traiter les peaux sèches comprenant l'administration orale, d'au moins une quantité efficace d'au moins deux microorganismes, dont au moins un est de type probiotique et est choisi parmi les *Lactobacillus johnsonii* (CNCM 1-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longue* (BB536), et leurs mélanges et/ou l'une de ses fractions et/ou l'un de ses métabolites en association avec une quantité efficace d'au moins un cation minéral divalent.

3. Procédé selon la revendication 1 ou 2, **caractérisé en ce qu'**il comprend l'administration d'au moins un cation minéral divalent sous la forme d'un sel différent d'un sulfate.

4. Procédé de traitement cosmétique pour prévenir et/ou traiter les peaux sensibles, comprenant l'administration topique, d'au moins une quantité efficace d'au moins un microorganisme probiotique choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM 1-3446), *Bifdobacterium longum* (BB536), et leurs mélanges et/ou l'une de ses fractions et/ou l'un de ses métabolites en association avec une quantité efficace d'au moins un cation minéral divalent sous la forme d'un sel différent d'un sulfate.

5. Procédé de traitement cosmétique pour prévenir et/ou traiter les peaux sèches, comprenant l'administration topique, d'au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifdobacterium adolescentis* (CNCM 1-2168), *Bifidobacterium longum* (CNCM I-2170), *Bifidobacterium lactis* (CNCM 1-3446), *Bifidobacterium longum* (BB536), et leurs mélanges et/ou l'une de ses fractions et/ou l'un de ses métabolites en association avec une quantité, efficace d'au moins un cation minéral divalent sous la forme d'un sel différent d'un sulfate.

6. Procédé selon l'une quelconque des revendications 1 à 5, **caractérisé en ce qu'**il comprend l'administration d'au moins un cation minéral divalent sous la forme d'un sel organique, anhydre ou hydraté, ou d'un complexe chélaté.

7. Procédé selon l'une quelconque des revendications 1, 3, 4 ou 6 **caractérisé en ce qu'**il comprend l'administration d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM 1-3446), *Bifidobacterium longum* (BB536), et leurs mélanges et/ou fractions et/ou métabolites de ceux-ci, différents.

8. Procédé selon l'une quelconque des revendications précédentes, **caractérisé en ce que** ledit ou lesdits microorganisme(s) probiotique(s) et/ou une de ses ou leurs fractions et/ou un de ses ou leurs métabolites est/sont formulé(s) dans un support physiologiquement acceptable, en une quantité équivalente à au moins 10³ ufc/g, notamment variant de 10⁵ à 10¹⁵ ufc/g, et en particulier de 10⁷ à 10¹² ufc/g de support.

9. Procédé selon l'une quelconque des revendications précédentes, **caractérisé en ce qu'**il comprend l'administration d'au moins deux cations minéraux divalents différents.

10. Procédé selon l'une quelconque des revendications précédentes, **caractérisé en ce que** ledit cation minéral divalent est un métal alcalino-terreux.

11. Procédé selon la revendication précédente, **caractérisé en ce que** ledit métal alcalino-terreux est choisi parmi le baryum, le calcium, le magnésium, le strontium, le béryllium et leurs mélanges.

12. Procédé selon l'une quelconque des revendications précédentes, **caractérisé en ce que** ledit cation est sous la forme d'un sel choisi parmi des carbonates, des bicarbonates, des gluconates, des glycérophosphates, des chlorures, des nitrates, des acétates, des hydroxydes, des oxydes, des sels d'α-hydroxyacides comme les citrates, tartrates, lactates et malates ou d'acides de fruits, des sels d'acides aminés comme les aspartates, arginates, et fumarates ou des sels d'acides gras comme les palmitates, oléates, caséinates et béhénates.

13. Procédé selon l'une quelconque des revendications précédentes; **caractérisé en ce que** ledit cation minéral divalent est sous la forme d'un sel choisi parmi les gluconates, citrates, lactates, chlorures et acétates de calcium, de strontium ou de magnésium et leurs mélanges.

14. Procédé selon l'une quelconque des revendications précédentes, **caractérisé en ce que** la teneur totale quotidienne en cation minéral divalent varie de 100 µg à 5 g, de 1 mg à 2 g, voire de 10 mg à 1,3 g.

15. Procédé selon l'une quelconque des revendications précédentes, **caractérisé en ce qu'**il associe au moins un sel de magnésium et/ou de calcium, notamment choisi parmi du gluconate, du lactate et/ou de citrate de magnésium et/ou de calcium et le nitrate de strontium, avec au moins une bactérie lactique choisie parmi les *Lactobacillus johnsonii* (CNCM I-1225) et *Lactobacillus paracasei* (CNCM I-2116).

16. Procédé selon l'une quelconque des revendications précédentes, **caractérisé en ce que** le microorganisme et/ou l'une de ses fractions et/ou l'un de ses métabolites est administré à raison de 5.10⁵ à 10¹³ ufc par jour et en particulier 10⁸ à 10¹¹ ufc par jour et que le cation minéral divalent est administré à des doses de l'ordre de 1 à 3000 mg par jour, et en particulier de 10 à 2000 mg par jour.

17. Procédé selon l'une quelconque des revendications précédentes, **caractérisé en ce que** l'association se présente sous la forme d'une composition cosmétique.

18. Procédé selon l'une quelconque des revendications 1 à 16, **caractérisé en ce que** l'association se présente sous la forme d'une composition alimentaire.

19. Utilisation cosmétique par voie orale, comme agent destiné à prévenir et/ou traiter les peaux sensibles d'une quantité efficace d'au moins un microorganisme probiotique choisi parmi les *Lactobacillus johnsonii* (CNCM 1-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent.

20. Utilisation cosmétique comme agent destiné à prévenir et/ou traiter les peaux sensibles, d'au moins une quantité efficace d'au moins un microorganisme probiotique choisi parmi les *Lactobacillus johnsonii* (CNCM 1-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins un cation minéral divalent sous la forme d'un sel différent d'un sulfate.

21. Utilisation cosmétique par voie orale comme agent destiné à prévenir et/ou traiter les peaux sèches d'une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM 1-2168), *Bifidobacterium longum* (CNCM I-2170), *Bifidobacterium lactis* (CNCM 1-3446), *Bifidobacterium longum* (BB536), et leurs mélanges et/ou l'une de ses fractions et/ou l'un de ses métabolites en association avec au moins un cation minéral divalent.

22. Composition destinée à une administration orale, comprenant au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM 1-2170), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent pour le traitement et/ou la prévention des peaux sèches choisies parmi la dermatite atopique et les ichtyoses.

23. Composition comprenant au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association avec au moins un cation minéral divalent sous la forme d'un sel différent d'un sulfate pour le traitement et/ou la prévention des peaux sèches choisies parmi la dermatite atopique et les ichtyoses.

24. Utilisation cosmétique comme agent destiné à prévenir et/ou traiter les peaux sèches d'au moins une quantité efficace d'au moins deux microorganismes, dont au moins un est de type probiotique, et est choisi parmi les *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins un cation minéral divalent sous la forme d'un sel différent d'un sulfate.

25. Utilisation selon l'une quelconque des revendications 19 à 24, **caractérisée en ce que** ledit ou lesdits microorganisme(s) est/sont tel(s) que défini(s) selon la revendication 8.

26. Utilisation selon la revendication 19 ou 20, **caractérisée en ce qu'**elle comprend en outre au moins un second microorganisme, notamment probiotique et/ou métabolites et/ou fractions, différent.

27. Utilisation selon l'une quelconque des revendications 19 à 26, **caractérisée en ce que** ledit cation est tel que défini en revendications 6 et 9 à 16.

28. Utilisation selon l'une quelconque des revendications 19 à 24, **caractérisée en ce qu'**il s'agit d'une composition ou d'une utilisation alimentaire, et en particulier destinée à la consommation humaine.

29. Utilisation selon l'une quelconque des revendications 22 ou 23, **caractérisée en ce qu'**il s'agit d'une composition dermatologique.

30. Composition cosmétique et/ou dermatologique, utile pour prévenir et/ou traiter les peaux sensibles et/ou sèches, comprenant dans un support physiologiquement acceptable au moins une quantité efficace d'au moins deux microorganismes probiotiques choisis parmi les *Lactobacillus johnsonii* (CNCM 1-1225), *Lactobacillus paracasei* (CNCM I-2116), *Bifidobacterium adolescentis* (CNCM I-2168), *Bifidobacterium longum* (CNCM 1-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins deux métaux alcalinoterreux sous la forme de sels organiques, anhydres ou hydratés ou de complexes chélatés.

31. Composition selon la revendication 30 **caractérisée en ce que** ledit métal alcalinoterreux est tel que défini en revendications 11 à 14.

32. Composition cosmétique et/ou dermatologique, utile pour prévenir et/ou traiter les peaux sensibles et/ou sèches, comprenant dans un support physiologiquement acceptable au moins une quantité efficace d'au moins deux microorganismes probiotiques choisis parmi les *Lactobacillus johnsonii* (CNCM 1-1225), *Lactobacillus paracasei* (CNCM I-2116), *Bifidobacterium adolescentis* (CNCM I-2168), *Bifidobacterium longum* (CNCM 1-2170), *Bifidobacterium lactis* (CNCM I-3446), *Bifidobacterium longum* (BB536), et leurs mélanges et/ou l'une de ses fractions et/ou l'un de ses métabolites en association à une quantité efficace d'au moins un métal alcalinoterreux sous la forme d'un sel choisi parmi, des bicarbonates, des glycérophosphates, des nitrates, des acétates, des hydroxydes, des sels d'α-hydroxyacides comme les citrates, tartrates, lactates et malates ou d'acides de fruits, des sels d'acides aminés comme les aspartates, arginates, et fumarates ou des sels d'acides gras comme les palmitates, oléates, caséinates et béhénates.

## Claims

1. Method .of cosmetic treatment, for preventing and/or treating sensitive skin, comprising the oral administration of at least an effective amount of at least one probiotic microorganism chosen from *Lactobacillus johnsonii* (CNCM I-1225), *Lactobacillus paracasei* (CNCM I-2116). *Bifidobacterium adolescentis* (CNCM I-2168), *Bifidobacterium longum* (CNCM I-2170), *Bifidobacterium lactic* (CNCM I-3446) and *Bifidobacterium longum* (BB536), and mixtures thereof, and/or a fraction thereof and/or a metabolite thereof, in combination with an effective amount of at least one divalent inorganic cation.

2. Method of cosmetic treatment for preventing and/or treading dry skin, comprising the oral administration of at least an effective amount of at least two microorganisms, at least one of which is of the probiotic type and is chosen from *Lactobacillus johsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM 1-3446) and *Bifidobacterium longum* (BB536), and mixtures thereof, and/or a fraction thereof and/or a metabolite thereof, in combination with an effective amount of at least one divalent inorganic cation.

3. Method according to Claim 1 or 2, **characterized in that** it comprises the administration of at least one divalent inorganic cation in the form of a salt other than a sulphate.

4. Method of cosmetic treatment for preventing and/or treating sensitize skin, comprising the topical administration of at least an effective amount of at least one probiotic microorganism chosen from *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM 1-3446) and *Bifidobacterium longum* (BB536), and mixtures thereof, and/or a fraction thereof and/or a metabolite thereof, in combination with an effective amount of at least one divalent inorganic cation in the form of a salt other than a sulphate.

5. Method of cosmetic treatment for preventing and/or treating dry skin, comprising the topical administration of at least an effective amount of at least two microorganisms, at least one of which is of the probiotic type and is chosen from *Lactobacillus johnsonii* (CNCM I-1225), *Lactobacillus paracasei* (CNCM I-2116), *Bifidobacterium adolescent* (CNCM 1-2168), *Bifidobacterium longum* (CNCM I-2170), *Bifidobacterium lactis* (CNCM 1-3446) and *Bifidobacterium longum* (*B*B536), and mixtures thereof, and/or a fraction thereof and/or a metabolite thereof, in combination with an effective amount of at least one divalent inorganic cation in the form of a salt other than a sulphate.

6. Method according to any one of Claims 1 to 5, **characterized in that** it comprise the administration of at least one divalent inorganic cation in the form of an anhydrous or hydrated organic salt, or of a chelated complex.

7. Method according to any one of Claims 1, 3, 4 or 6, **characterized in that** it comprises the administration of at least two microorganisms, at least one of which is of the probiotic type and is chosen from *Lactobacillus johnsonii* (CNCM 1-1225), *Lactobacillus paracasei* (CNCM I-2116), *Bifidobacterium adolescents* (CNCM I-2168), *Bifidobacterium longum* (CNCM I-2170), *Bifidobacterium lactis* (CNCM I-3446) and *Bifidobacterium longum* (BB536), and mixtures thereof, and/or fractions and/or metabolites thereof, that are different.

8. Method according to any one of the preceding claims, **characterized in that** said probiotic microorganism(s) and/or a fraction thereof and/or a metabolite thereof, is/are formulated in a physiologically acceptable carrier in an amount equivalent to at least 10³ cfu/g, especially ranging from 10⁵ to 10¹⁵ cfu/g, and in particular from 10⁷ to 10¹² cfu/g of carrier.

9. Method according to any one of the preceding claims, **characterized in that** it comprises the administration of at least two different divalent inorganic cations.

10. Method according to any one of the preceding claims, **characterized in that** said divalent inorganic cation is an alkaline earth metal.

11. Method according to the preceding claim, **characterized in that** said alkaline earth metal is chosen from barium, calcium, magnesium, strontium and beryllium, and mixtures thereof.

12. Method according to any one of the preceding claims, **characterized in that** said cation is in the form of a salt chosen from carbonate, bicarbonates, gluconates, glycerophosphates, chlorides, nitrates, acetates, hydroxides, oxides, α-hydroxy acid salts such as citrates, tartrates, lactates and malates, or of fruit acids, amino acid salts such as aspartates, arginates and fumarates, or fatty acid salts such as palpitates, oleates, caseinates and behenates.

13. Method according to any one of the preceding claims, **characterized in that** said divalent inorganic cation is in the form of a salt chosen from calcium gluconates, citrates, lactates, chlorides and acetates, strontium gluconates, citrates, lactates, chlorides and acetates or magnesium gluconates, citrates, lactates, chlorides and acetates, and mixtures thereof.

14. Method according to any one of the preceding claims, **characterized in that** the total daily content of divalent inorganic cation ranges from 100 µg to 5 g, from 1 mg to 2 g, or even from 10 mg to 1.3 g,

15. Method according to any one of the preceding claims, **characterized in that** it combines at least one magnesium salt and/or calcium salt, in particular chosen from magnesium gluconate, lactate and/or citrate and/or calcium gluconate, lactate and/or citrate and strontium nitrate, with at least one lactic acid bacterium, in particular chosen from *Lactobacillus johnsonii* (CNCM 1-1225) and *Lactobacillus paracasei* (CNCM I-2116)

16. Method according to any one of the preceding claims, **characterized in that** the microorganism and/or a fraction thereof and/or a metabolite thereof is administered at a rate of 5 × 10⁵ to 10¹³ cfu per day, and in particular 10⁸ to 10¹¹ cfu per day, and **in that** the divalent inorganic cation is administered at doses of the order of 1 to 3000 mg per day, and in particular of 10 to 2000 mg per day.

17. Method according to any one of the preceding claims, **characterized in that** the combination is in the form of a cosmetic composition.

18. Method according to any one of Claims 1 to 16, **characterized in that** the combination is in the form of a food composition.

19. Oral cosmetic use, as an agent intended for preventing and/or treating sensitive skin, of an effective amount of at least one probiotic microorganism chosen from *Lactobacillus johnsonii* (CNCM I-1225), *Lactobacillus paracasei* (CNCM I-2116), *Bifidobacterium adolescentis* (CNCM I-2168), Bifidobacterium *longum* (CNCM I-2170), *Bifidobacterium lactic* (CNCM I-3446) and *Bifidobacterium longum* (BB536), and mixtures thereof, and/or a fraction thereof and/or a metabolite thereof, in combination with at least one divalent inorganic cation.

20. Cosmetic use, as an agent intended for preventing and/or treating sensitive skin, of at least an effective amount of at least one probiotic microorganism chosen from *Lactobacillus johnsonii* (CNCM I-1225), *Lactobacillus paracasei* (CNCM I-2116), *Bifidobacterium adolescents* (CNCM I-2168), *Bifidobacterium longum* (CNCM I-2170), *Bifidobacterium* lactic (CNCM 1-3446) and *Bifidobacterium longum* (BB536), and mixtures thereof, and/or a fraction thereof and/or a metabolite thereof, in combination with an effective amount of at least one divalent inorganic cation in the form of a salt other than a sulphate.

21. Oral cosmetic use, as an agent intended for preventing and/or treating dry skin, of at least an effective amount of at least two microorganisms, at least one of which is of the probiotic type and is chosen from *Lactobacillus johnsonii* (CNCM 1-1225), *Lactobacillus paracasei* (CNCM I-2116), *Bifidobacterium adolescentis* (CNCM 1-2168), *Bifidobacterium longum* (CNCM I-2170), *Bifidobacterium lactis* (CNCM 1-3446) and *Bifidobacterium longum* (BB536), and mixtures thereof, and/or a fraction thereof and/or a metabolite thereof, in combination with at least one divalent inorganic cation.

22. Composition intended for oral administration, comprising at least an effective amount of at least two microorganisms, at least one of which is of the probiotic hype and is chosen from *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3446) and *Bifidobacterium longum* (BB536), and mixtures thereof, and/or a fraction thereof and/or a metabolite thereof, in combination with at least one divalent inorganic cation, for treating and/or preventing dry skin chosen from atopic dermatitis and ichthyoses.

23. Composition comprising at least an effective amount of at least two microorganisms, at least one of which is of the probiotic type and is chosen from *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM 1-3446) and *Bifidobacterium longum* (BB536), and mixtures thereof, and/or a fraction thereof and/ora metabolite thereof, in combination with at least one divalent inorganic cation in the form of a salt other than a sulphate, for treating and/or preventing dry skin chosen from atopic dermatitis and ichthyoses,

24. Cosmetic use, as an agent intended for preventing and/or treating dry skin, of at least an effective amount of at least two microorganisms at least one of which is of the probiotic type and is chosen from *Lactobacillus johnsonii* (CNCM I-1225), *Lactobacillus paracasei* (CNCM I-2116), *Bifidobacterium adolescent* (CNCM I-2168), *Bifidobacterium longum* (CNCM I-2170), *Bifidobacterium lactic* (CNCM 1-3446) and *Bifidobacterium longum* (BB536), and mixtures thereof and/or a fraction thereof and/or a metabolite thereof, in combination with an effective amount of at least one divalent inorganic cation in the form of a salt other than a sulphate.

25. Use according to any one of Claims 19 to 24, **characterized in that** said microorganism(s) is/are as defined in claim 8.

26. Use according to Claim 19 or 20, **characterized in that** it also comprises at least a second, different, microorganism, in particular probiotic microorganism, and/or metabolites and/or fractions.

27. Use according to any one of Claims 19 to 26, **characterized in that** said cation is as defined in Claims 6 and 9 to 16.

28. Use according to any one of Claims 19 to 24, **characterized in that** it involves a food composition or a dietary use in particular intended for human consumption.

29. Use according to either of Claims 22 and 23, **characterized in that** the composition is a dermatological composition.

30. Cosmetic and/or dermatological composition, that is useful for preventing and/or treating sensitive and/or dry skin, comprising, in a physiologically acceptable carrier, at least an effective amount of at least two probiotic microorganisms chosen from *Lactobacillus johnsonii* (CNCM I-1225), *Lactobacillus paracasei* (CNCM I-2116), *Bifidobacterium adolescentis* (CNCM I-2168), *Bifidobacterium longum* (CNCM I-2170), *Bifidobacterium lactis* (CNCM I-3445) and *Bifidobacterium longum* (BB536), and mixtures thereof and/or a fraction thereof and/or a metabolite thereof, in combination with an effective amount of at least two alkaline earth metals in the form of anhydrous or hydrated organic salts, or of chelated complexes,

31. Composition according to Claim 30, **characterized in that** said alkaline earth metal is as defined in Claims 11 to 14.

32. Cosmetic and/or dermatological composition, that is useful for preventing and/or treating sensitive and/or dry skin, comprising, in a physiologically acceptable carrier, at least an effective amount of at least two probiotic microorganisms chosen from *Lactobacillus johnsonii* (CNCM I-1225), *Lactobacillus parecasei* (CNCM I-2116), *Bifidobacterium adolescentis* (CNCM I-2168), *Bifidobacterium longum* (CNCM I-2170), *Bifidobacterium lactic* (CNCM 1-3446) and *Bifidobacterium longum* (BB536), and mixtures thereof and/or a fraction thereof and/or a metabolite thereof, in combination with an effective amount of at least one alkaline earth metal in the form of a salt chosen from bicarbonates, glycerophosphates, nitrate, acetates, hydroxides, α-hydroxy acid salts, such as citrates, tartrates, lactates and malates, or of fruit acids, amino acid salts, such as aspartates, arginates and fumarates, or fatty acid salts such as palmitates, oleates, caseinates and behenates..

## Patentansprüche

1. Kosmetisches Behandlungsverfahren zur Verhütung und/oder Behandlung empfindlicher Haut, umfassend die orale Verabreichung von mindestens einer wirksamen Menge von mindestens einem probiotischen Mikroorganismus, ausgewählt aus *Lactobacillus johnsonii* (CNCM-I-1225), *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BB536) und ihren Gemischen und/oder von einer seiner Fraktionen, und/oder von einem seiner Metaboliten, in Kombination mit einer wirksamen Menge von mindestens einem zweiwertigen mineralischen Kation.

2. Kosmetisches Behandlungsverfahren zur Verhütung und/oder Behandlung trockener Haut; umfassend die orale Verabreichung von mindestens einer wirksamen Menge von mindestens zwei Mikroorganismen, wovon mindestens einer vomprobiotischen Typ ist und ausgewählt ist aus *Lactobacillus johnsonii* (CNCM-I-1225). *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BB536) und ihren Gemischen, und/oder von einer ihrer Fraktionen und/oder von einem ihrer Metaboliten in Kombination mit einer wirksamen Menge von mindestens einem zweiwertigen mineralischen Kation.

3. Verfahren nach Anspruch 1 oder 2. **dadurch** gekennezeichnet, dass es die Verabreichung von mindestens einem zweiwertigen mineralischen Kation in der Form eines von einem Sulfat verschiedenen Salzes umfasst,

4. Kosmetisches Behandlungsverfahren zur Verhütung und/oder Behandlung empfindlicher Haut, umfassend die topische Verabreichung von mindestens einer wirksamen Menge von mindestens einem probiotischen Mikroorganismus, ausgewählt aus *Lactobacillus johnsonii* (CNCM-I-1225), *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BB536) und ihren Gemischen, und/oder von einer seiner Fraktionen und/oder von einem seiner Metaboliten in Kombination mit einer wirksamen Menge von mindestens einem zweiwertigen mineralischen Kation in der Form eines von einem Sulfat verschiedenen Salzes.

5. Kosmetisches Behandlungsverfahren zur Verhütung und/oder Behandlung trockener Haut, umfassend die topische Verabreichung von mindestens einer wirksamen Menge von mindestens zwei Mikroorganismen, wovon mindestens einer vom probiotischen Typ ist und ausgewählt ist aus *Lactobacillus johnsonii* (CNCM-I-1225), *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BBS36) und ihren Gemischen, und/oder von einer ihrer Fraktionen und/oder von einem ihrer Metaboliten in Kombination mit einer wirksamen Menge von mindestens einem zweiwertigen mineralischen Kation in der Form eines von einem Sulfat verschiedenen Salzes.

6. Verfahren nach einem der Anspruche 1 bis 5, **dadurch gekennzeichnet, dass** es die Verabreichung von mindestens einem zweiwertigen mineralischen Kation in der Form eines organischen, wasserfreien oder hydratisierten Salzes oder eines Chelatkomplexes umfasst.

7. Verfahren nach einem der Anspruche 1, 3, 4 oder 6, **dadurch gekennzeichnet, dass** es die Verabreichung von mindestens zwei Mikroorganismen umfasst, wovon mindestens einer vom probiotischen Typ ist und ausgewählt ist aus *Lactobacillus johnsonii* (CNCM-I-1225), *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BB536) und ihren verschiedenen Gemischen, und/oder Fraktionen und/oder Metaboliten davon.

8. Verfahren nach einem der vorhergehenden Ansprüche, **dadurch gekennzeichnet, dass** der (die) probiotische(n) Mikroorganismus(en) und/oder eine von seinen oder ihren Fraktionen und/oder einer von seinen oder ihren Metaboliten in einem physiologisch verträglichen Träger in einer Menge entsprechend mindestens 10³ ufc/g, insbesondere variierend von 10⁵ bis 10¹⁵ ufc/g, und ganz besonders von 10⁷ bis 10¹² ufc/g von Träger, formuliert wird (werden).

9. Verfahren nach einem der vorhergehenden Ansprüche, **dadurch gekennzeichnet, dass** es die Verabreichung von mindestens zwei mineralischen zweiwertigen verschiedenen Kationen umfasst.

10. Verfahren nach einem der vorhergehenden Ansprüche, **dadurch gekennzeichnet, dass** das zweiwertige mineralische Kation ein Erdalkalimetall ist.

11. Verfahren nach dem vorhergehenden Anspruch, **dadurch gekennzeichnet, dass** das Erdalkalimetall aus Barium, Calcium, Magnesium, Strontium, Beryllium und ihren Gemischen ausgewählt ist.

12. Verfahren nach einem der vorhergehenden Ansprüche, **dadurch gekennzeichnet, dass** das Kation in der Form eines Salzes vorhanden ist, ausgewählt aus Carbonaten, Bicarbonaten, Gluconaten, Glycerophosphaten, Chloriden, Nitraten, Acetaten, Hydroxiden, Oxiden, Salzen von α-Hydroxysäuren, wie Citrate, Tartrate, Lactate, und Malate, oder von Fruchtsäuren, Salzen von Aminosäuren, wie Aspartate, Arginate und Fumaraten oder Salzen von Fettsäuren, wie Palmitate, Oleate, Caseinate und Behenate.

13. Verfahren nach einem der vorhergehenden Ansprüche, **dadurch gekennzeichnet, dass** das zweiwertigen, mineralische Kation in der Form eines Salzes vorhanden ist, das ausgewählt ist aus Gluconaten, Citraten, Lactaten, Chloriden und Acetaten von Calcium, Strontium oder von Magnesium und ihren Gemischen.

14. Verfahren nach einem der vorhergehenden Ansprüche, **dadurch gekennzeichnet, dass** der tägliche Gesamtgehalt an zweiwertigem, mineralischem Kation von 100 µg bis 5 g, von 1 mg bis 2 g, und sogar von 10 mg bis 1,3 g variiert.

15. Verfahren nach einem der vorhergehenden Ansprüche, **dadurch gekennzeichnet, dass** es mindestens ein Magnesium- und/oder Calciumsalz, insbesondere ausgewählt aus Gluconat, Lactat und/oder Citrat von Magnesium und/oder Calcium, und Strontiumnitrat mit mindestens einem Milchsäurebacterium, ausgewählt aus *Lactobacillus johnsonii* (CNCM-I-1225) und *Lactobacillus paracasei* (CNCM-I-2116), kombiniert.

16. Verfahren nach einem der vorhergehenden Ansprüche, **dadurch gekennzeichnet, dass** der Mikroorganismus und/oder eine von seinen Fraktionen und/oder einer von seinen Metaboliten in einem Verhältnis von 5.10⁵ bis 10¹³ ufc pro Tag und insbesondere 10⁸ bis 10¹¹ ufc pro Tag verabreicht wird und **dadurch**, dass das zweiwertige mineralische Kation in Dosen in der Größenordnung von 1 bis 3000 mg pro Tag, und insbesondere 10 bis 2000 mg pro Tag verabreicht wird.

17. Verfahren nach einem der vorhergehenden Ansprüche, **dadurch gekennzeichnet, dass** die Kombination in der Form einer kosmetischen Zusammensetzung vorhanden ist.

18. Verfahren nach einem der Ansprüche 1 bis 16, **dadurch gekennzeichnet, dass** die Kombination in der Form einer Nahrungsmittelzusammensetzung vorliegt.

19. Kosmetische orale Anwendung einer wirksamen Menge von mindestens einem probiotischen Mikroorganismus, ausgewählt aus *Lactobacillus johnsonii* (CNCM-I-1225), *Lactobacillus paracasei* (CNCM-I-2116), *Bifidobacterium adolescentis* (CNCM-I-2168), *Bifidobacterium longum* (CNCM-I-2170), *Bifidobacterium lactis* (CNCM-I-3446), *Bifidobacterium longum* (BB536) und ihren Gemischen, und/oder einer seiner Fraktionen und/oder eines seiner Metaboliten in Kombination mit mindestens einem zweiwertigen mineralischen Kation als Mittel zur Verhütung und/oder Behandlung von empfindlicher Haut.

20. Kosmetische Anwendung mindestens einer wirksamen Menge von mindestens einem probiotischen Mikroorganismus, ausgewählt aus *Lactobacillus johnsonii* (CNCM-I-1225), *Lactobacillus paracasei* (CNCM-I-2116), *Bifidobacterium adolescentis* (CNCM-I-2168), B*ifidobacterium longum* (CNCM-I-2170), *Bifidobacterium lactis* (CNCM-I-3446), *Bifidobacterium longum* (BB536) und ihren Gemischen, und/oder von einer seiner Fraktionen und/oder eines seiner Metaboliten in Kombination mit einer wirksamen Menge mindestens eines zweiwertigen mineralischen Kations in der Form eines von Sulfat verschiedenen Salzes als Mittel zur Verhütung und/oder Behandlung von empfindlicher Haut.

21. Kosmetische orale Anwendung einer wirksamen Menge von mindestens zwei Mikroorganismen, wovon mindestens einer vom probiotischen Typ ist und ausgewählt ist aus *Lactobacillus johnsonii* (CNCM-I-1225), *Lactobacillus paracasei* (CNCM-I-2116), B*ifidobacterium adolescentis* (CNCM-I-2168), B*ifidobacterium longum* (CNCM-I-2170), B*ifidobacterium lactis* (CNCM-I-3446), B*ifidobacterium longum* (BB536) und ihren Gemischen, und/oder von eines ihrer Fraktionen und/oder eines ihrer Metaboliten in Kombination mit mindestens einem zweiwertigen mineralischen Kation als Mittel zur Verhütung und/oder Behandlung von trockener Haut.

22. Zur oralen Verabreichung bestimmte Zusammensetzung, umfassend mindestens eine wirksame Menge von mindestens zwei Mikroorganismen, wovon mindestens einer vom probiotischen Typ ist und ausgewählt ist aus *Lactobacillus johnsonii* (CNCM-I-1225), *Lactobacillus paracasei* (CNCM-I-2116), B*ifidobacterium adolescentis* (CNCM-I-2168), B*ifidobacterium longum* (CNCM-I-2170), B*ifidobacterium lactis* (CNCM-I-3446), B*ifidobacterium longum* (BB536) und ihren Gemischen, und/oder einer ihrer Fraktionen und/oder eines ihrer Metaboliten in Kombination mit mindestens einem zweiwertigen mineralischen Kation zur Behandlung und/oder Verhütung von trockener Haut, die aus atopischer Dermatitis und Ichtyosen ausgewählt ist.

23. Zusammensetzung, umfassend mindestens eine wirksame Menge von mindestens zwei Mikroorganismen, wovon mindestens einer vom probiotischen Typ ist und ausgewählt ist aus *Lactobacillus johnsonii* (CNCM-I-1225); *Lactobacillus paracasei* (CNCM-I-2116), B*ifidobacterium adolescentis* (CNCM-I-2168), B*ifidobacterium longum* (CNCM-I-2170), B*ifidobacterium lactis* (CNCM-I-3446), B*ifidobacterium longum* (BB536) und ihren Gemischen, und/oder einer ihrer Fraktionen und/oder einem ihrer Metaboliten in Kombination mit mindestens einem zweiwertigen mineralischen Kation in der Form eines von Sulfat verschiedenen Salzes zur Behandlung und/oder Verhütung von trockener Haut, die aus atopischer Dermatitis und Ichtyosen ausgewählt ist.

24. Kosmetische Verwendung mindestens einer wirksamen Menge von mindestens zwei Mikroorganismen, wovon mindestens einer vom probiotischen Typ ist und ausgewählt ist aus *Lactobacillus johnsonii* (CNCM-I-1225), *Lactobacillus paracasei* (CNCM-I-2116), B*ifidobacterium adolescentis* (CNCM-I-2168), B*ifidobacterium longum* (CNCM-I-2170), B*ifidobacterium lactis* (CNCM-I-3446), B*ifidobacterium longum* (BB536) und ihren Gemischen, und/oder einer ihrer Fraktionen und/oder eines ihrer Metaboliten in Kombination mit mindestens einem zweiwertigen mineralischen Kation in der Form eines von Sulfat verschiedenen Salzes als Mittel zur Verhütung und/oder Behandlung von trockener Haut.

25. Verwendung nach einem der Ansprüche 19 bis 24, **dadurch gekennzeichnet, dass** der (die) Mikroorganismus (Mikroorganismen) wie in Anspruch 8 definiert ist (sind).

26. Verwendung nach Anspruch 19 oder 20, **dadurch gekennzeichnet, dass** sie weiterhin mindestens einen zweiten verschiedenen, insbesondere probiotischen Mikroorganismus, und/oder Metaboliten und/oder Fraktionen umfasst.

27. Verwendung nach einem der Ansprüche 19 bis 26, **dadurch gekennzeichnet, dass** das Kation wie in den Ansprüchen 6 und 9 bis 16 definiert ist.

28. Verwendung nach einem der Ansprüche 19 bis 24, **dadurch gekennzeichnet, dass** es sich um eine Zusammensetzung oder um eine Nahrungsmittelverwendung, und insbesondere bestimmt zum menschlichen Verzehr, handelt.

29. Verwendung nach einem der Ansprüche 22 oder 23, **dadurch gekennzeichnet, dass** es sich um eine dermatologische Zusammensetzung handelt.

30. Zur Verhütung und/oder Behandlung von empfindlicher und/oder trockener Haut geeignete kosmetische und/oder dermatologische Zusammensetzung, umfassend in einem physiologisch verträglichen Träger von mindestens einer wirksamen Menge von mindestens zwei probiotischen Mikroorganismen, die ausgewählt sind aus *Lactobacillus johnsonii* (CNCM-I-1225), *Lactobacillus paracasei* (CNCM-I-2116), B*ifidobacterium adolescentis* (CNCM-I-2168), B*ifidobacterium longum* (CNCM-I-*2170),* B*ifidobacterium lactis* (CNCM-I-3446), B*ifidobacterium longum* (BB536) und ihren Gemischen, und/oder von einer ihrer Fraktionen und/oder von einem ihrer Metaboliten in Kombination mit einer wirksamen Menge mindestens zwei Erdalkalimetallen in der Form von organischen, wasserfreien oder hydratisierten Salzen oder von Chelatkomplexen.

31. Zusammensetzung nach Anspruch 30, **dadurch gekennzeichnet, dass** das Erdalkalimetall wie in den Ansprüchen 11 bis 14 definiert ist.

32. Zur Verhütung und/oder Behandlung von empfindlicher und/oder trockener Haut geeignet kosmetische und/oder dermatologische Zusammensetzung, umfassend in einem physiologisch verträglichen Träger mindestens eine wirksame Menge von mindestens zwei probiotischen Mikroorganismen, die ausgewählt sind aus *Lactobacillus johnsonii* (CNCM-I-1225), *Lactobacillus paracasei* (CNCM-I-2116), B*ifidobacterium adolescentis* (CNCM-I-2168), B*ifidobacterium longum* (CNCM-I-2170), B*ifidobacterium lactis* (CNCM-I-3446), B*ifidobacterium longum* (BB536) und ihren Gemischen, und/oder von einer ihrer Fraktionen und/oder von einem ihrer Metaboliten in Kombination mit einer wirksamen Menge mindestens eines Erdalkalimetalls in der Form eines Salzes, ausgewählt aus Bicarbonaten, Glycerophosphaten, Nitraten, Acetaten, Hydroxiden, Salzen von α-Hydroxysäuren, wie Citrate, Tartrate, Lactate, und Malate, oder von Fruchtsäuren, Salzen von Aminosäuren, wie Aspartate, Arginate und Fumaraten, oder Salzen von Fettsäuren, wie Palmitate, Oleate, Caseinate und Behenate.
